# EUROPEAN PATENT SPECIFICATION

(11) **EP 4 392 989 B1**
(45) Date of publication and mention of the grant of the patent: **30.09.2026**
(21) Application number: 22859658.1
(22) Date of filing: 25.08.2022
(51) Int. Cl.: G16H 50/70, A61B 34/10, A61F 2/30, A61F 2/44, G06N 20/00, G06T 17/20

(54) **A METHOD AND MEDICAL IMPLANT**
VERFAHREN UND MEDIZINISCHES IMPLANTAT
PROCÉDÉ ET IMPLANT MÉDICAL

(30) Priority: 25.08.2021 AU 2021902738
(43) Date of publication of application: 03.07.2024
(73) Proprietor: 3Dmorphic PTY Ltd, Matraville, NSW 2036 (AU)
(72) Inventor: PARR, William Chase Harington, Matraville, New South Wales 2036 (AU)
(74) Representative: Murgitroyd & Company
(86) International application number: PCT/AU2022/050998
(87) International publication number: WO 2023/023771

(56) References cited:
- WO-A1-2009/025783
- WO-A1-2010/099359
- WO-A1-2015/057898
- WO-A1-2017/127887
- US-A- 5 514 180
- US-A1- 2007 043 442
- WU, K ET AL.: "Development and selection of Asian-specific humeral implants based on statistical atlas: towards planning minimally invasive surgery", INTERNATIONAL JOURNAL OF COMPUTER ASSISTED RADIOLOGY AND SURGERY, vol. 10, 2015, pages 1333 - 1345, XP035524266, DOI: 10.1007/s11548-014-1140-7
- WU, K ET AL.: "Statistical atlas-based morphological variation analysis of the asian humerus: Towards consistent allometric implant positioning", INTERNATIONAL JOURNAL OF COMPUTER ASSISTED RADIOLOGY AND SURGERY, vol. 10, 2015, pages 317 - 327, XP035455982, DOI: 10.1007/s11548-014-1084-y
- SINGH TELVINDERJIT; PARR WILLIAM CHASE HARINGTON; CHOY WEN JIE; BUDIONO GIDEON RICHARD; MAHARAJ MONISH; MATHIS XAVIER; PHAN KEVIN;: "Three-Dimensional Morphometric Analysis of Lumbar Vertebral End Plate Anatomy", WORLD NEUROSURGERY, ELSEVIER, AMSTERDAM, NL, vol. 135, 3 December 2019 (2019-12-03), AMSTERDAM, NL , XP086070760, ISSN: 1878-8750, DOI: 10.1016/j.wneu.2019.11.158
- MOBBS RALPH J., PARR WILLIAM C.H., CHOY WEN JIE, MCEVOY AIDAN, WALSH WILLIAM R., PHAN KEVIN: "Anterior Lumbar Interbody Fusion Using a Personalized Approach: Is Custom the Future of Implants for Anterior Lumbar Interbody Fusion Surgery?", WORLD NEUROSURGERY, ELSEVIER, AMSTERDAM, NL, vol. 124, 1 April 2019 (2019-04-01), AMSTERDAM, NL , pages 452 - 458.e1, XP093040828, ISSN: 1878-8750, DOI: 10.1016/j.wneu.2018.12.144
- CLOGENSEN, M ET AL.: "A statistical shape model of the human second cervical vertebra", INTERNATIONAL JOURNAL OF COMPUTER ASSISTED RADIOLOGY AND SURGERY, vol. 10, 2015, pages 1097 - 1107, XP035506227, DOI: 10.1007/s11548-014-1121-x

## Description

### CROSS REFERENCE TO RELATED APPLICATION

This application claims priority to Australian Provisional Patent Application No 2021902738 filed 25 August 2021.

### FIELD OF THE INVENTION

The invention relates to a method of developing a shape for a medical implant. In particular, the invention relates, but is not limited, to a method based on data from a variety of patients along with a medical implant and system.

### BACKGROUND TO THE INVENTION

Reference to background art herein is not to be construed as an admission that such art constitutes common general knowledge in Australia or elsewhere.

Poorly fitting medical implants can cause a number of issues for patients. For example, poorly fitting medical implants may cause stress hot-spots and point loads. They may also result in further surgical alteration of a patient's (bony) anatomy to accommodate the implant. During surgery this can lead to: i) increase in blood loss as bone is highly vascularised; ii) prolong surgical procedures; iii) increase risk of infection; and iv) further adverse effects due to prolonged anaesthesia.

Removing bone to accommodate poorly fitting implants is also problematic as the removed surface is typically strong, stiff cortical bone (stiffness modulus, E ~ 10-20GPa). Once removed, softer underlying cancellous bone (E ~200-400MPa) is exposed to, and loaded by, the implanted device. Loading of weaker cancellous bone is particularly problematic in spinal fusion surgery as the predominant loading scenario is compression from vertebral bodies to the interbody implant. Fatigue cycles of an interbody device to the vertebrae construct, in which the device is loading the weaker cancellous bone, has been shown to increase the likelihood of the device sinking into the bone (subsidence).

Device subsidence is problematic for interbody implant surgeries as it reduces the interbody height restoration achieved by the implantation of the device. If a device continues to subside with repeated loading, the surgery may not achieve the biomechanical stability necessary for bone to grow onto the device (osseointegration) or for bone-to-bone fusion to occur, which are important endpoints to achieve for long term treatment of the disease and relief of clinical symptoms for the patient. Severe subsidence may also necessitate revision surgery.

A potential option for creating better fitting spinal interbody devices, or medical implants, is to design and manufacture devices that are custom-made specifically for the patient that they are intended to be used in. This option will likely give a device that fits the patient's anatomy very well, but is disruptive to current manufacturing and distribution supply chains and mechanisms.

When a custom-made device is not an option, there is still a need for a better fitting generic, or 'off-the-shelf', device. Current interbody device designs, particularly for arthrodesis (fusion) devices, are often symmetrical. This has advantages in terms of ease of design, manufacture, testing and implantation as it does not matter which way up the devices are used and means there is no chance of a device being implanted 'the wrong way up'. However, the simple geometric shapes of current 'off-the-shelf' devices contribute to poor implant fit with patient anatomy. Furthermore, 'off-the-shelf' implants are also typically scaled isometrically, which can lead to poor fits between different patients. Therefore, there is a need for better fitting implantable devices, particularly for spinal interbody devices.

With the above in mind, WU, K. et al. published 'Development and selection of Asian-specific humeral implants based on statistical atlas: towards planning minimally invasive surgery' in 2014. WU, K. et al. relates to a fuzzy C-means clustering method to better search for matching implants from a database. A contribution of WU, K. et al. is the development of Asian, gender and cluster-specific implants based on shape features and selection of best fit implants. 'Statistical atlas-based morphological variation analysis of the Asian humerus: Towards consistent allometric implant positioning', also published by WU, K. et al. in 2015, makes a similar contribution.

WO 2015/057898 discloses a method of constructing a mass-customized orthopedic implant by identifying features in a statistical atlas population of bones and generating an electronic design file for a mass-customized orthopedic implant. In order to extract anatomical variations across a common anatomy, input anatomy data is compared to a common frame of reference across a population, commonly referred to as a template 3D model or anatomical 3D template model. In this regard, WO 2015057898 aims to replace bones.

WO 2010/099359 relates to orthopaedic implants and, more specifically, to methods and devices utilised to design orthopaedic implants and orthopaedic jigs for use with joint replacement and revision procedures. In this regard, WO 2010/099359 is similar to WO 2015/057898.

US 20070043442 relates generally to an implantable device for promoting the fusion of adjacent bony structures, and a method of using the same. US 20070043442 describes digitising a bone structure and analysing the shape between adjacent vertebrae from various people.

US 5514180 relates to anterior prosthetic intervertebral devices which may be inserted in the vertebral disc spaces resulting from the removal of diseased or damaged intervertebral discs. Various vertebrae may be digitised in US 5514180 and a Hardy multiquadric is used to find a best fit surface.

WO 2009/025783 relates to a method for anatomical analysis and joint implant design. An exemplary statistical atlas may be created by choosing a bone model with average shape characteristics. In a similar manner to WO 2010/099359 and WO 2015/057898, WO 2009/025783 is primarily directed towards replacing bones.

WO 2017/127887 relates to producing a digital model of a customised device where files are imported and modified to generate a model of a customised device. WO 2017/127887 is a previous application of the Applicant.

The following documents also relate to medical devices but their relevance varies:
a) SINGH, T. et al, 'Three-Dimensional Morphometric Analysis of Lumbar Vertebral End Plate Anatomy', World Neurosurgery, March 2020, 134, pages 321-332;
b) MOBBS, R. et al, 'Anterior Lumbar Interbody Fusion Using a Personalized Approach: Is Custom the Future of Implants for Anterior Lumbar Interbody Fusion Surgery?', World Neurosurgery, 2019, 124, pages 452-458; and
c) CLOGENSEN, M. et al, 'A statistical shape model of the human second cervical vertebra', International Journal of Computer Assisted Radiology and Surgery, 2015, 10, pages 1097-1107.

### SUMMARY OF INVENTION

The present invention relates to a method as defined in claim 1, a medical implant as defined in claim 14 and a medical implant system as defined in claim 15. Preferred embodiments of the invention are set forth in the dependent claims. method is disclosed including:
using a model to assist in defining one or more outer surfaces of a medical implant, the model being based on a statistical analysis of data, associated with an intended location of a medical implant, from multiple patients; and
producing the medical implant based on the one or more outer surfaces,
wherein:
the data includes information associated with patients of different sizes; and
the step of using the model includes retrieving a statistical shape based on the statistical analysis of the data;
   the statistical shape includes at least two surfaces and a space therebetween that assists in defining the one or more surfaces of the medical implant.

In an embodiment, the data includes information associated with patients making up at least a standard deviation about an average size for a population.

In an embodiment, the data includes information associated with patients of different height.

In an embodiment, the intended location is the spine. In an embodiment, the intended location includes a location associated with two adjacent vertebral bodies.

In an embodiment, using the model includes: retrieving a statistical shape based on the statistical analysis of the data; and defining the one or more outer surfaces of a medical implant based on the statistical shape.

In an embodiment, the model assists in providing an outline or device footprint contour(s) that aids in generating the one or more outer surfaces.

In an embodiment, the statistical shape includes a surface that assists in defining the one or more outlines/device footprint contours of the medical implant.

In an embodiment, the outline or device footprint contour(s) are directly related to the one or more surfaces.

In an embodiment, the step of using the model includes using a statistical relationship established from the data to retrieve a statistical shape associated with the intended location of the medical implant.

In an embodiment, the statistical relationship includes an equation relating to a line of best fit through the data.

In an embodiment, the statistical relationship includes allometric scaling.

In an embodiment, the statistical relationship includes isometric scaling.

In an embodiment, the statistical relationship includes a relationship formed with the data having three dimensional data. In an embodiment, the three dimensional data is in the form of geometric data/information.

In an embodiment, the statistical analysis of the data is in more than two dimensions.

In an embodiment, a matrix decomposition is performed on the data as part of developing the model.

In an embodiment, the matrix decomposition is performed on a covariance matrix.

In an embodiment, the covariance matrix includes geometry data, size and/or an associated height.

In an embodiment, the geometry data includes x,y,z co-ordinates.

In an embodiment, a singular value decomposition is performed on the covariance matrix.

In an embodiment, an eigen decomposition is performed on the covariance matrix which forms the bases of a principal components analysis.

In an embodiment, the dimensionality of the data is reduced into a smaller subset. In an embodiment, the dimensionality of the data is reduced by at least one dimension.

In an embodiment, the smaller subset includes subset dimensions containing shape information.

In an embodiment, the subset dimensions are linear and form axes.

In an embodiment, scores are assigned to the data to assist in analysing shape variation. In an embodiment, the scores are assigned by the matrix decomposition.

In an embodiment, the scores relate to positions on subset dimension axes.

In an embodiment, the statistical analysis includes creating an n-dimensional shape space. In an embodiment, the n-dimensional shape space is defined by all, or some, of the subset dimension axes.

In an embodiment, scores on each of the n-dimension axes of the shape space contribute to identifying discrete shapes.

In an embodiment, using the model includes determining a confidence interval of data within a defined criteria.

In an embodiment, the confidence interval is sampled in a manner to create a suitable cohort of medical implants.

In an embodiment, the confidence interval is evenly sampled across its region to create the suitable cohort of medical implants.

In an embodiment, the confidence interval is unevenly sampled across its region, according to the frequency distribution of the data, to create the suitable cohort of medical implants.

In a further embodiment, in response to identify a predetermined shape variance, a shape space can be used to create a range of statistical shapes capturing the predetermined shape variance at discrete intervals.

In an embodiment, in response to the medical implant being for a specific region, the data is refined to focus on that specific region.

In an embodiment, the method further comprises including one or more device parameters to assist in defining the one or more outer surfaces of the medical implant.

In an embodiment, the one or more device parameters are separate from the data.

In an embodiment, the one or more device parameters are based on one or more predetermined parameters.

In an embodiment, the one or more predetermined parameters are set by an external specification.

In an embodiment, the external specification includes: a surgeon specification; and/or published specifications in scientific literature.

In an embodiment, the one or more device parameters are defined by ASTM F2077 or one or more other standards.

In an embodiment, the one or more device parameters include maximum or minimum device dimensions, maximum or minimum device angles and/or a fastening portion.

In an embodiment, the step of producing the medical implant based on the one or more outer surfaces includes producing two surfaces that are asymmetric.

In an embodiment, defining the one or more outer surfaces includes warping an initial shape to a target shape.

In an embodiment, the target shape assists in accommodating greater contact surface area with the statistical shape.

In an embodiment, the one or more outer surfaces includes more than one outer surface.

In another form, a medical implant is disclosed comprising:
a body having:
   one or more outer surfaces configured to engage with an intended location of the medical implant,
wherein:
   the one or more outer surfaces are based on a statistical analysis of data:
      associated with the intended location of the medical implant; and
      from multiple patients; and
   the one or more outer surfaces are established from a statistical relationship derived from the data whereby the statistical relationship allows a statistical shape to be established, the statistical shape includes at least two surfaces and a space therebetween that assists in defining the one or more outer surfaces of the medical implant.

In an embodiment, the data and intended location of the medical implant is herein as described.

In an embodiment, the one or more outer surfaces are established from a statistical relationship derived from the data whereby the statistical relationship allows a statistical shape to be established, the statistical shape being configured to complement the one or more outer surfaces.

In an embodiment, the one or more outer surfaces are associated with a line of best fit through the data.

In an embodiment, the one or more outer surfaces form part of an allometric relationship when compared to a set of other associated allometric medical implant(s).

In an embodiment, the one or more outer surfaces are associated with a predetermined confidence interval of the data. In an embodiment, the confidence interval is anywhere between 1% to 99%, 25% to 99%, 25% to 95%, 50% to 99%, 50% to 95%, 75% to 99% or 75% to 95%. In an embodiment, the confidence interval is 50%, 75% 95% or 99%.

In an embodiment, the one or more outer surfaces derived from the outlines/device footprint contours includes at least two surfaces that are asymmetric.

In an embodiment, the medical implant includes a height to assist with engagement with one or more interfacing instruments. In an embodiment, the interfacing instruments includes an inserter and/or integral screw.

In an embodiment, one or more device parameters assist in defining the one or more outer surfaces.

In an embodiment, the one or more device parameters are separate from the data.

In an embodiment, the one or more device parameters are based on one or more predetermined parameters set by an external specification.

In an embodiment, the medical implant complies with ASTM F2077. In an embodiment, the medical implant complies with height requirement(s) as defined in ASTM F2077.

In an embodiment, the body is enclosed in a packaging. In an embodiment, the packaging engaging the body is sterile.

In an embodiment, the one or more outer surfaces includes more than one outer surface.

In another form, a medical implant system is disclosed including:
multiple medical implants having one or more outer surfaces configured to engage with an intended location of each medical implant,
wherein:
   the one or more outer surfaces are based on a statistical analysis of data:
   associated with the intended locations of the medical implants; and
   from multiple patientsof different sizes; and
   the one or more outer surfaces are established from a statistical relationship derived from the data whereby the statistical relationship allows a statistical shape to be established, the statistical shape includes at least two surfaces and a space therebetween that assists in defining the one or more outer surfaces of the medical implant.

In an embodiment, the medical implants are herein as described.

In an embodiment, the medical implants relate to a statistical relationship established from the data.

In an embodiment, the medical implants follow a size related relationship.

In an embodiment, the medical implants follow an allometric relationship.

In an embodiment, aspects of the medical implants follow a relationship determine by a line of best fit through the data.

In an embodiment, the medical implants fall within a confidence interval associated with the data.

In an embodiment, the medical implants are substantially evenly spread over the confidence interval to provide a suitable cohort of devices for surgery.

In an embodiment, the medical implants are inter-replaced during surgery to find a suitable fit with the intended surgical location of a specific patient.

In an embodiment, one or more device parameters assist in defining the one or more outer surfaces.

In an embodiment, the one or more device parameters are separate from the data.

In an embodiment, the one or more device parameters are based on one or more predetermined parameters set by an external specification.

In an embodiment, the medical implants comply with ASTM F2077.

In an embodiment, the one or more outer surfaces includes more than one outer surface.

Further features and advantages of the present invention will become apparent from the following detailed description.

### BRIEF DESCRIPTION OF THE DRAWINGS

By way of example only, preferred embodiments of the invention will be described more fully hereinafter with reference to the accompanying figures, wherein:
Figure 1 illustrates a flow diagram of a method for developing the shape of implant devices for a medical implant system, according to an embodiment of the invention;
Figure 2 illustrates a perspective view of a cervical spine;
Figure 3 illustrates a graph showing regression analysis of different cervical interbody disc spaces;
Figure 4 illustrates a comparison of medical implants, according to an embodiment of the invention;
Figure 5 illustrates a statistical plot of cervical end plate shape scores;
Figure 6 illustrates cervical interbody statistical shapes corresponding to discrete points shown in Figure 5;
Figure 7 illustrates scatterplots of shape scores on three axes for endplate pairs;
Figure 8 illustrates a shape space associated with Figure 7;
Figure 9 illustrates mean lumbar spine vertebral endplate surface areas across all interbody spaces;
Figure 10 illustrates statistical shape models of lumbar intervertebral body spaces at the L1-2 and L2-3 levels (as noted in Figure 9);
Figure 11 illustrates an anterior-superior-left view of smaller and larger lateral lumbar interbody fusion devices corresponding to a size and shape of the smaller and larger interbody spaces (as shown in Figure 10);
Figure 12 illustrates a left view two interbody fusion devices between two statistical end plate shapes;
Figure 13 illustrates an implant device, based on statistical shapes, combined with other design requirements according to an embodiment of the invention;
Figure 14 illustrates implant devices, based on statistical shapes, combined with other design requirements;
Figure 15 illustrates further implant devices, based on statistical shapes, combined with other design requirements; and
Figure 16 illustrates a perspective view of an interbody device in an interbody space defined by superior and inferior endplate statistical shapes.

### DETAILED DESCRIPTION OF THE DRAWINGS

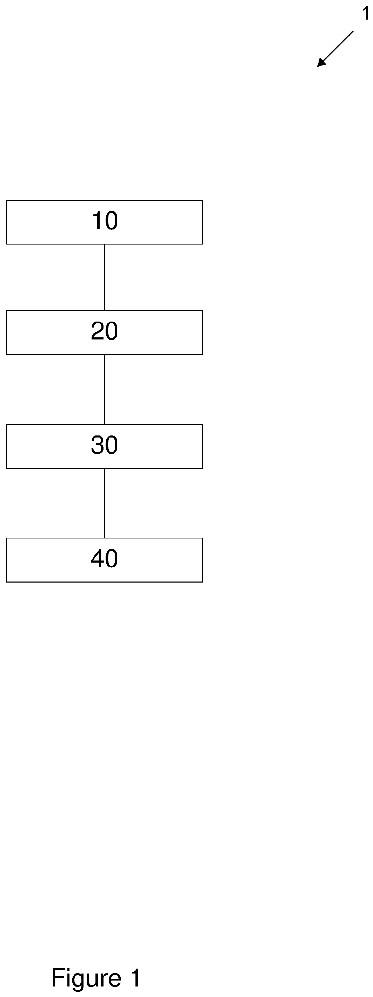

Figure 1 illustrates a method 1 of producing one or more medical implants, according to an embodiment of the invention. The method 1 includes a first step 10 of using one or more models to assist in defining one or more outer surfaces of a (generic) medical implant. As further detailed below, the models may be bivariate or multivariate. The models assist in providing a (scaling) relationship based on a statistical analysis of data, associated with an intended location of the medical implant, from multiple patients. The relationships may take the form of, for example, surface area scaling or more complex three dimensional (3D) shape scaling.

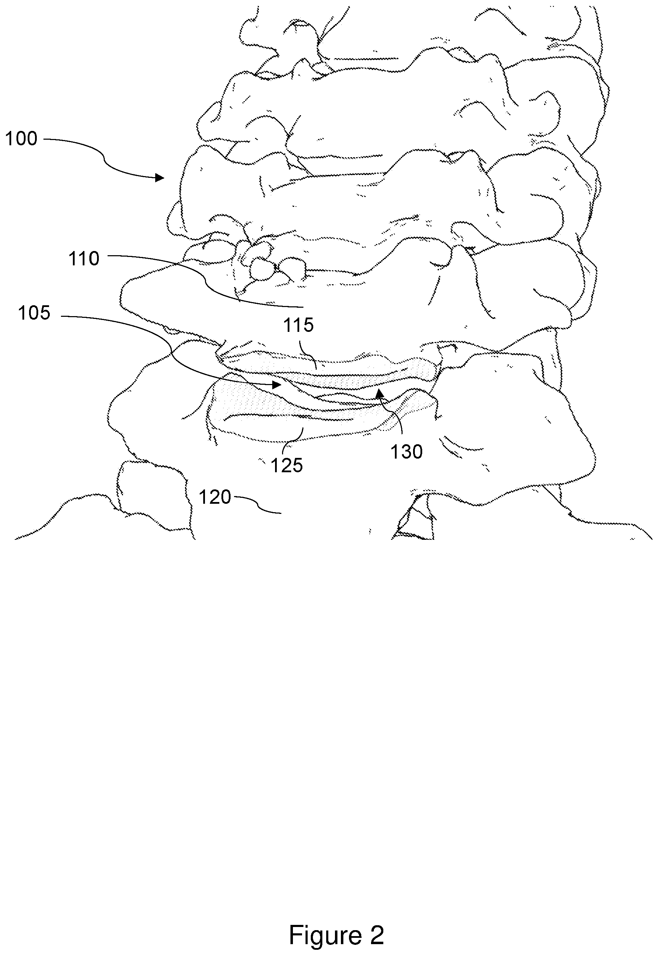

To further illustrate step 10, Figure 2 illustrates a perspective view of a cervical spine 100 showing an (C6-7) interbody space 105. The interbody space 105 is located between a (C6) vertebral body 110 and a (C7) vertebral body 120. The interbody space 105 is at least partly defined by a superior endplate 115 of the vertebrae 110 and an inferior end plate 125 of vertebrae 120. Collapse in the height of the interbody space 105, as a result of degenerative disc disease (for example), can lead to bone-to-bone rubbing of the interbody joint space and compression of the adjacent spinal cord 130 and nerve roots.

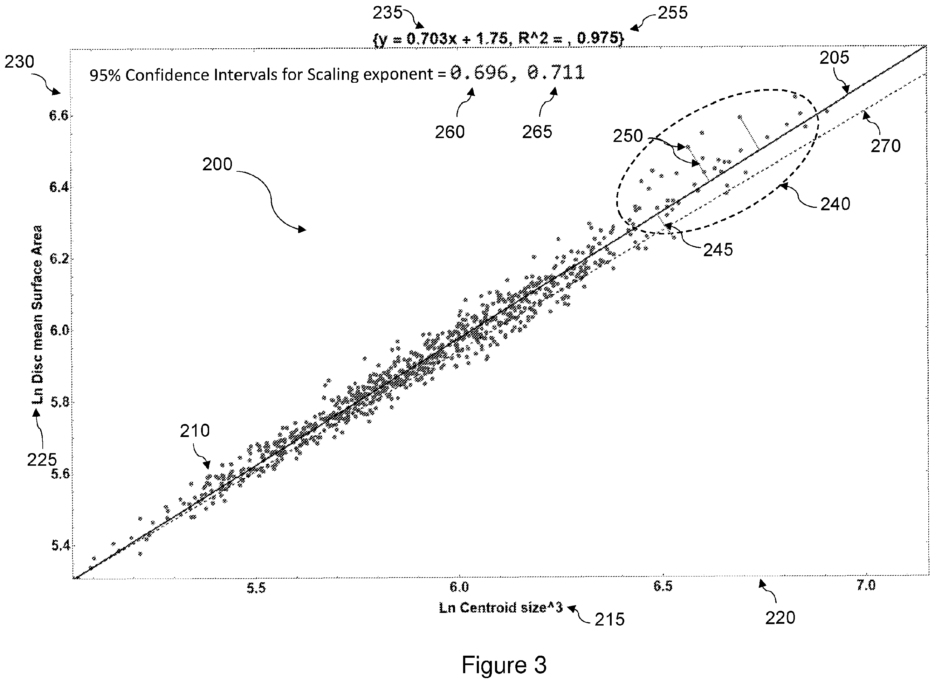

To develop a suitable model, Figure 3 illustrates a scatterplot of data 200 associated with interbody disc spaces 105 from a variety of people. That is, data relating to 780 separate interbody disc spaces was used in the analysis. The graph in Figure 3 shows a regression analysis of the natural log (Ln) of centroid size (^3) 215 along the x-axis 220. Centroid size is the arithmetic mean point/vertex vector magnitude of spine segment interbody shapes. In other words, it is a measurement of overall size. Ln of disc mean surface area 225 is plotted along the y-axis 230 and is defined as the arithmetic mean of the surface areas of the superior 115 and inferior 125 endplates defining each interbody disc space.

A best fit line 205 (or regression line) is plotted through the data points 210. This provides a relationship 235 in the form of y=0.703x + 1.75. The exponent (0.703) of this relationship 235 indicates that the associated medical devices should be scaled allometrically, not isometrically. That is, isometric relationships provide an exponent of 0.667. This can be established by appreciating the scaling factors of area (x²) and volume (x³) to maintain an isometric relationship (x^{2/3} where 2/3 = 0.667). In any case, it is important to note that the 95% minimum 260 and maximum 265 confidence intervals also do not include the 0.667 exponent. This indicates a significant difference between the empirically observed line, based on the data set, and the 0.667 dashed line 270 for isometry. Furthermore, the exponent confidence intervals are narrow as the R^2 value 255 is very high (ie, close to 1).

With the above in mind, isometric scaling for this particular data / patient anatomy (which is common practice) also becomes more problematic for larger individuals. As indicated by dashed ellipse line 240, there are more points 210 above the line 205 than below it. These points 210 above the line 205 generally have greater residuals 250 (the length of the line 250 from the point to the regression line 205) than the residual 245 for points below the regression line. This data therefore indicates that for larger individuals, isometric relationships do not suitably represent their particular needs and following an isometric relationship is unlikely to create a suitable implant. Rather, allometric scaling of the implant contact surfaces would more suitably result in larger devices being more tailored to larger patients (ie, they would be wider when scaled to the same anterior-posterior depth) whilst also improving the fit for smaller patients. It will also be appreciated that the exponent above may vary depending on the data set being used for the intended location of the medical implant. For example, the exponent may reflect either positive or negative allometry or isometry.

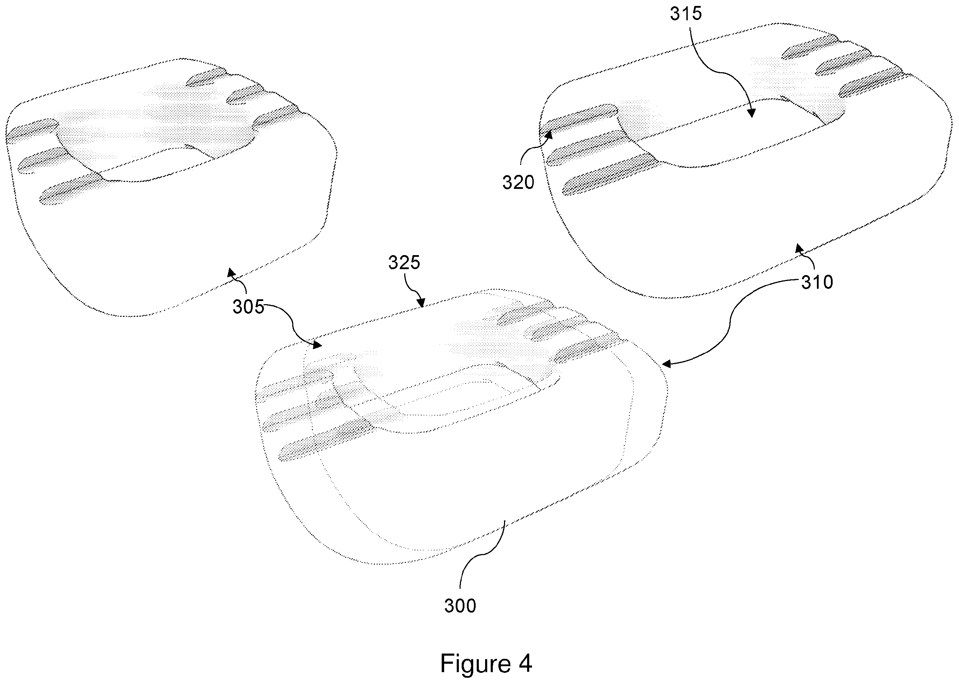

To illustrate the effect of the above scaling, Figure 4 illustrates a flat sided, zero angle interbody device designs 300 with graft windows 315 and anti-expulsion teeth 320. Allometric scaling of device-anatomy interface contact surface area results in larger devices 310 being wider than smaller devices 305 when the devices are scaled to the same anterior-posterior depth and height. This demonstrates the effects of size related allometric shape change on device design.

To this end, with relationship 235, a model is established that can be used to suitably predict a more ideal implant shape based on size of a patient. Refining the model to produce a discrete number of implants that will service a cohort of patients, as a medical implant system of generic devices, is further discussed below. In the present embodiment however, to define the outer surfaces of the medical implant in step 10, the relationship 235 is used to retrieve approximate surface areas/shapes of the endplates 115, 125 based on patient size. Patient size may include weight, height and/or body mass. Following this, the (negative) space between the endplates 115, 125 can be used to define curvatures of surfaces forming at least part of the body of the medical implant. Typically, outlines/device footprint contours are generated from the model that correlate, at least in part, to the surfaces of the implant. It will therefore be appreciated that the medical implant would then suitably complement the statistical shapes of the endplates 115, 125.

Whilst the model developed from the information in Figure 3 is effective, different parameters can have different scaling exponents to one another within the same biological entity. For example, linear measurements (eg, depth vs width), surface area, contour curvature and so forth can scale differently. This leads to more complex 3D shape modelling and scaling. The scaling detailed below is allometric but, based on the present disclosure, it will be appreciated that (for example) isometric scaling may result and this will be dictated by the data set and relationship uncovered. Furthermore, whilst modelling is discussed in relation to the spine, other areas of the patient / sample population may be used.

For interbody shapes, such as interbody space 105, the preferred method to establish a relationship is by examining the complex 3D shapes of pairs of endplates 115, 125. Other extracted parameters (eg, curvature arc radii, linear metrics, surface areas etc.) are available but a preferred 3D shape analysis will be discussed below.

At the outset, analysis of paired endplate three-dimensional morphology allows for covarying shape traits, types, modes, or components to be identified in one analysis, rather than decomposing the full 3D shapes into certain parameters. Separately, bivariate or multivariate analyses can be used on the one or more data sets to identify relationships. These relationships can then be used as inputs to create a computer aided design (CAD) file of the implant.

By way of example, geometric morphometric landmark-based methods can be utilised to achieve full 3D shape analysis of interbody space shapes where the landmarks form the control points/vertices that define the endplate surfaces. Matrix decomposition can be performed on the covariance matrix of the x,y,z coordinates and additional parameters, such as weights, of the control points/vertices. Matrix decomposition may include singular value decomposition, eigen decomposition which can form the bases of principal components analysis, or other non-orthogonal decompositions can be used depending on the analysis goals. In orthogonal decompositions, such as singular value/eigen decomposition, shape traits identified within a mode/on an axis are correlated/covary but are uncorrelated with shape traits identified in the other orthonormal shape modes/axes. Such decompositions result in the identification of different types/modes/components of shape variation. In other words, statistical shapes can be created through decomposition of a covariance matrix, where the (mathematical) data in the covariance matrix consists of the x,y,z 3D coordinates of points/vertices defining the endplate surfaces. These points/vertices can be surface control points (in the case of Bezier surfaces, Non-uniform rational Bezier spline, NURBS surfaces, or Catmull-Clark type subdivision, sub-d surfaces) or the vertices defining corners of planar triangles (triangulated tessellation surface representation).

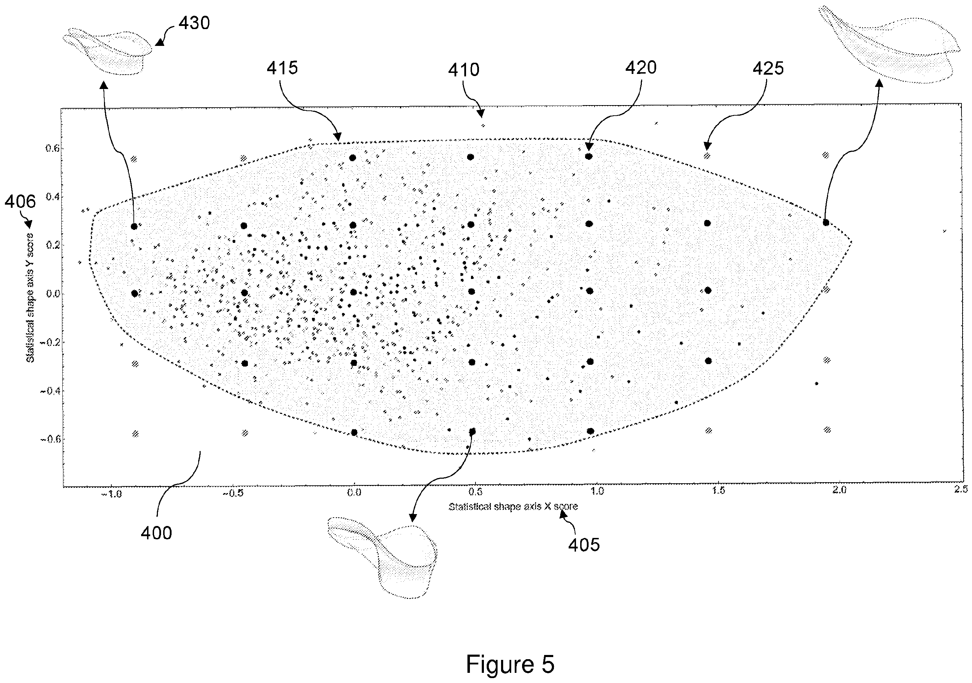

To illustrate the results of a matrix decomposition, Figure 5 shows shape scores associated with two axes resulting from a Principal Component (PC) analysis. The superior and inferior endplate surfaces for each interbody space are analysed together so that shape characteristics that covary between the superior and inferior interbody space endplates are captured by the analysis. PC analysis is a dimensionality-reduction method that can be used to identify the main types/modes/components of variation in large datasets. This method reduces the dimensionality of large data sets by capturing the majority of variation in a dataset in a few components or axes. More specifically, specimens making up the analysis, or subsequently entered into the statistical shape space/model, can be assigned a score along each axis based on their shape. This means that a bivariate shape space of PC1 against PC2 can approximate anywhere between ~30 and ~70% of the total shape variation, meaning a PC1-PC2 shape space approximation of an individual specimen's shape will be 30-70% accurate to the actual specimen shape.

Turning more specifically to Figure 5, a 2-dimensional scatterplot 400 of statistical shape scores for 780 cervical endplate pairs is shown. In other words, scores from different types/modes/axes of statistical shape variation can be plotted against one another in a bivariate plot. X 405 and Y 406 axis scores can be translated into statistical shapes (eg, shape 430). Accordingly, each grey point 410 and black point 420 represents a different discrete shape within the shape space continuum. The superior and inferior endplate surfaces for each interbody space are analysed together so that shape characteristics that covary between the superior and inferior interbody space endplates are captured by the analysis.

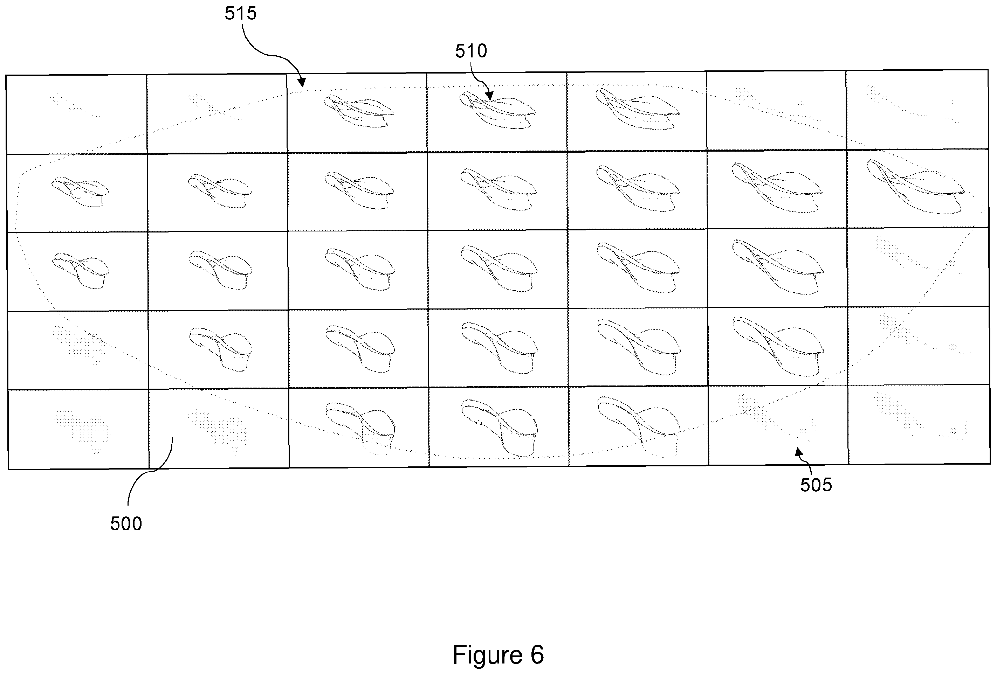

At step 20 in Figure 1, a select group of statistical shapes are defined based on the model. At this point, these statistical shapes of the anatomy, which can be used to define implant parameters and/or shape. This select group suitable covers a predetermine quantile of the population. In this regard, the shape defined by dotted line 415 (ie, the convex hull), in Figure 5, illustrates a 99% polytope quantile. That is, it captures 99% of the axis 1 and 2 shape variation of the sample. In further embodiments, this could vary anywhere between (for example) 1% to 99%. In the plot 400, the entire shape space is substantially sampled uniformly at discrete points 420. Accordingly, making implants that complement the statistical shapes at the discrete points 420 assists in ensuring that a cohort (or select group) of devices are made that suitably complement a defined quantile. The statistical shapes may not necessarily correspond to a data point and this is the benefit of using the present models - suitable shapes can be retrieved to cover the defined quantile. To further illustrate this, Figure 6 shows a representation 500 of the cervical interbody statistical shapes corresponding to the discrete points shown in 400. Line drawings 510 are shapes that fall within the 99% polytope quartile convex hull (dashed line 515). The dashed line 515 corresponds to the dotted line 415. Translucent shaded shapes 505 represent interbody shapes outside the 99% quantile. The shapes outside the 99% quartile 505 are not used to define interbody device shapes in the present embodiment.

For practical purposes, such as identifying shapes commonly occurring within a population so that a device that fits these shapes can be designed, the total shape space may be subsampled to capture different proportions (eg, 50%, 75%, 95%, 99%) of the total sample shape variance. With this in mind, whilst the shape space in Figure 5 is 2-dimensional, the method (or step 10) can be extended to n-dimensional shape space, with scores on each of the n-dimension axes of the shape space contributing to the discrete shapes. This allows a much greater proportion of the shape variation present in a dataset to be captured and represented in the discrete statistical shapes generated.

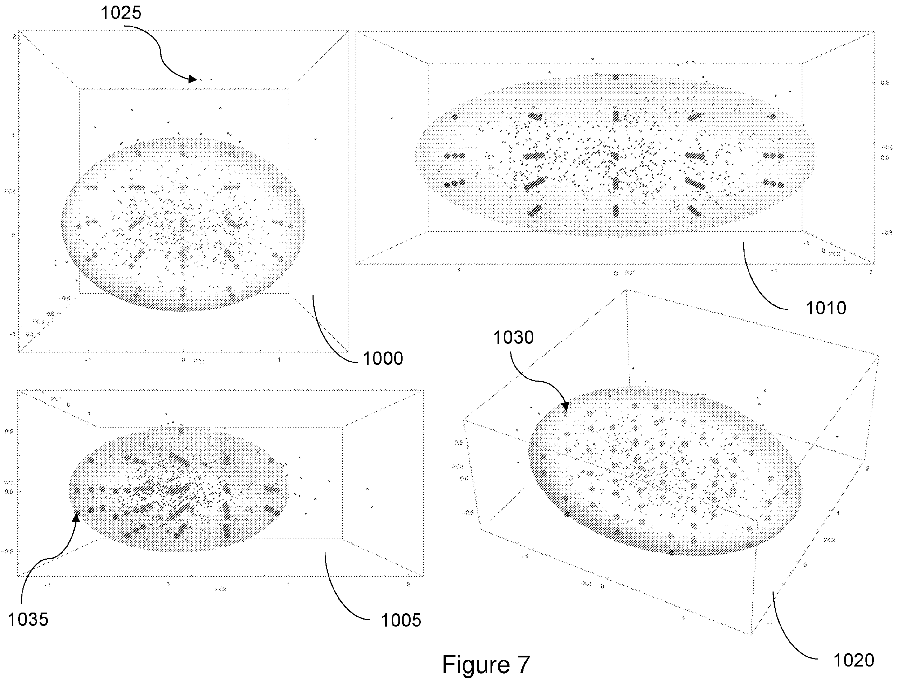

For example, Figure 7 illustrates scatterplots of scores on three out of the n-dimensions of shape space resulting from analysis of single level cervical corpectomy endplate pairs. Before discussing Figure 7 in detail, it should be noted that the scope of the sample used to construct the covariance matrix and entered into the shape analysis can be narrowed or widened depending on the device design goals. For instance, to create an interbody device range potentially suited to any level in the cervical spine, the sample should contain all levels of the spine (eg, C2-3, C3-4, C4-5, C5-6, C6-7). In some circumstances, there can be advantages to using the data in different ways. By way of another example, if the goal is to design a one level corpectomy implant suitable for the cervical spine, then the pairings of the endplates entered into the shape analysis could be: C2 caudal endplate-C4 cranial endplate (C2-C4); C3-C5, C4-C6; and C5-C7. The modes/components/types of statistical shapes, as well as the shape space, identified by analyses of these pairings differ to those defined in the analysis of (disc) interbody spaces discussed above.

Turning to Figure 7, a decomposition was performed of the covariance matrix of xyz triangulated vertices of single level cervical corpectomy spaces (ie, C2-C4, C3-C5, C4-C6 and C5-C7). This is part of a PC analysis (in a similar manner to Figure 5). In Figure 7, the same plot from different viewpoints is shown (ie, PC1 scores vs PC 2 scores in graph 1000; PC2 vs PC3 in graph 1005; PC1 vs PC3 in graph 1010; and PC1 vs PC2 vs PC3 in graph 1020). Individual scores for the corpectomy endplate shapes are shown by small points 1025. The 95% confidence interval (CI) ellipsoid is shown as space 1030. The shape space was sampled at discrete points (as part of step 20), with scores falling within the 95% CI ellipsoid shown as larger black points 1035. Each of these discrete points has an x, y, z coordinate within the 3D shape space scatterplots shown. These coordinates represent scores along each x (PC1), y (PC2), z (PC3) axis. Each axis defines different shape characteristics, or components. In PC analysis, but not all shape analyses, the shapes captured on each axis are un-correlated with one another. In a similar manner to the analysis in Figure 5, points 1035 are selected at discrete uniform locations. This allows a selection of shapes that will ultimately assist in defining the shapes of generic implants that will more suitably serve a population. The points 1035 are substantially equidistant from one another to assist with providing ample coverage in the 95%CI. As will be appreciated, other, such as economic, factors will guide how many points 1035 should be included as this will correspond to how many generic devices are manufactured for the implant system.

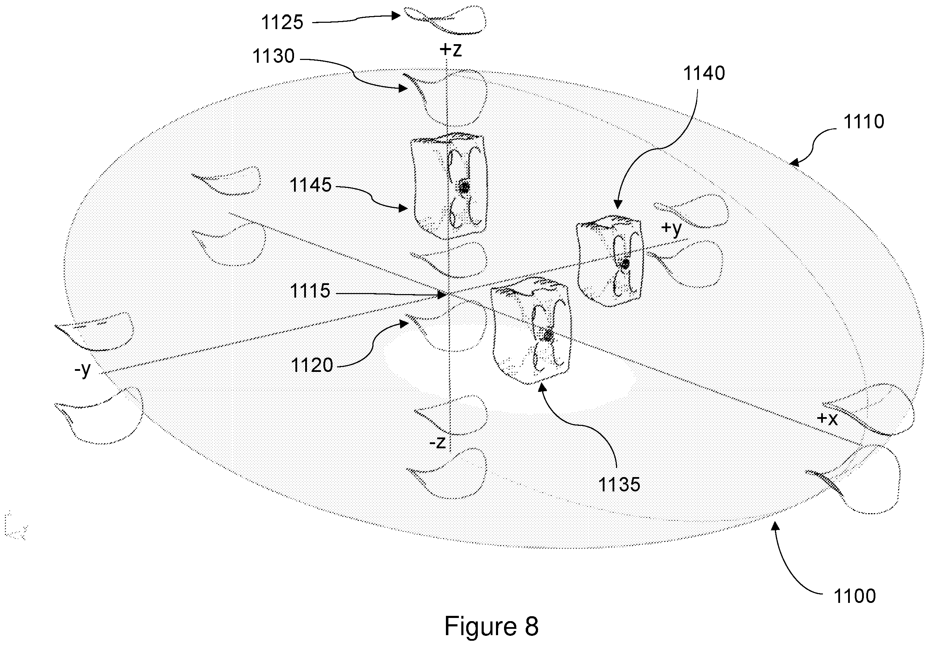

Overall, CI regions (or sub-spaces) within the whole shape space can be used, as part of step 20, to reduce the total amount of implant designs that are manufactured as shapes outside of the CI regions do not generally require devices designed for them. To further demonstrate this, Figure 8 illustrates space shape 1030 from Figure 7 as shape 1100 with x, y and z axes. That is, the 95% CI is an ellipsoid in three dimensions as shown by shape 1110. The discrete shapes defined by the points of intersection of the axes with the shape 1110 are shown along with the mean shape 1120 which is the shape defined at the origin of the shape space (x,y,z 0,0,0 in this 3 dimensional example). Each statistical shape is represented by a superior endplate 1125 and inferior endplate 1130. Any point within the shape space can be represented by cartesian (x,y,z) coordinates that correspond to axis 1,2,3 scores. These scores can be used to generate a statistical shape at this point, which can be used to design the implant device. That is, the statistical shapes assists in defining one or more outlines/device footprint contours. These outlines/device footprint contours aid in generating the surfaces of the implant. Shown are three cervical corpectomy devices at points on the x axis 1135, y axis 1140 and z axis 1145. The combination of axes used to define the statistical shapes to design the implants do not necessarily have to include all axes identified by the decomposition (for example a subset of three out of many are shown in figure 8) or be in order (in figure 8 xyz axes correspond to PC123, but could, just as well, correspond to PC1,5,8). In the present example, the x-axis is correlated with the distance between the endplates. For an expandable, or stackable, device system, there may be an advantage in not using this axis in favour of another axis of shape variation.

In some circumstances, there may be advantages to breaking up an anatomical region into different segments, as part of step 10, and creating separate statistical shapes / models for the different segments. For example, vertebral endplate surface area generally increases down the length of the spine (cranial to caudal). This is due to each subsequent (caudal progression) interbody space bearing proportionally greater amounts of the body's total mass. However, there can be level specific differences in endplate surface areas within an anatomical region of the spine. This means that the progression in surface area increase is not always linear, meaning that either: i) other structures are bearing some of the load, reducing the force through particular levels; ii) the biomechanical constraints, or function, of levels differ from one-another; or iii) some levels are relatively overloaded, with stresses closer to safety margins. For instance, the L5-S1 interbody space is notable in this regard, having much lower endplate surface areas than would be predicted by a straightforward linear increase in endplate surface areas (cranial-caudal).

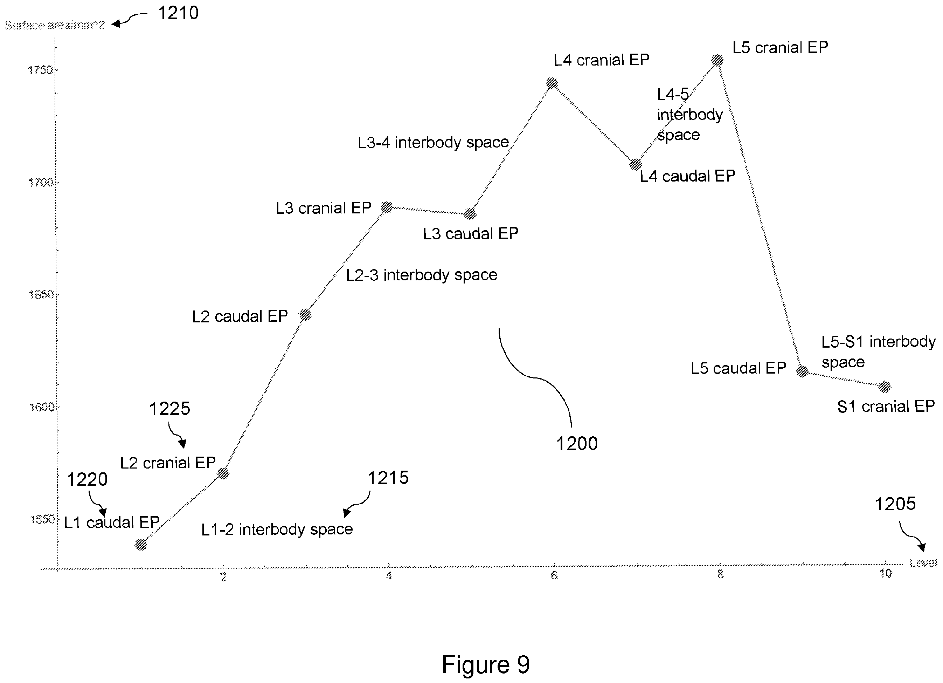

The reduced size of the L5-S1 interbody space may contribute to the relative prevalence of this level among lumbar degenerative disc disease patients that are indicated for surgery as lower endplate surface areas may result in higher pressures / stresses in the vertebral endplates and/or disc compared to other interbody levels. Higher stresses/strains may increase the risk of disc damage as, all other factors being equal, peak loads will result in stresses / strains closer to the yield limits of disc soft tissue components (e.g. collagen fibres in the annular layers). To this end, Figure 9 illustrates line graph 1200 of mean (of an adult population sample) lumbar spine vertebral endplate surface areas (mm^2) 1210 across all lumbar interbody spaces (L1-L2, L2-3, L3-4, L4-5, L5-S1), or levels 1205. Each point represents the surface area of an endplate, with opposing endplates 1220 and 1225 defining a disc/interbody space 1215. Note that there is a general increase in lumbar endplate surface area between L1 caudal endplate to the L5 cranial endplate. Also of note is that there are 3 different trends shown: i) L1-2 and L2-3 endplates sequentially increase in surface area; ii) in L3-L4 and L4-L5 the superior endplate of the disc space has a lower surface area than the inferior endplate of the disc space above; and iii) the L5-S1 disc space endplates have lower surface areas than all other disc spaces except L1-2.

Accordingly, in lumbar interbody fusion procedures, a lateral approach is rarely, if ever, used to instrument the L5-S1 level as the pelvis obscures direct access to the space. Therefore, if the goal is to create statistical shapes for use in designing lateral approach devices, the L5-S1 level may be excluded. In addition, an anterior approach is less favoured in comparison to a lateral approach for higher lumbar levels. Therefore, the lumbar spine interbody spaces can be grouped into (T12-L1), L1-2, L2-3 for design of lateral approach devices (eg, LLIF, OLIF, XLIF fusion devices).

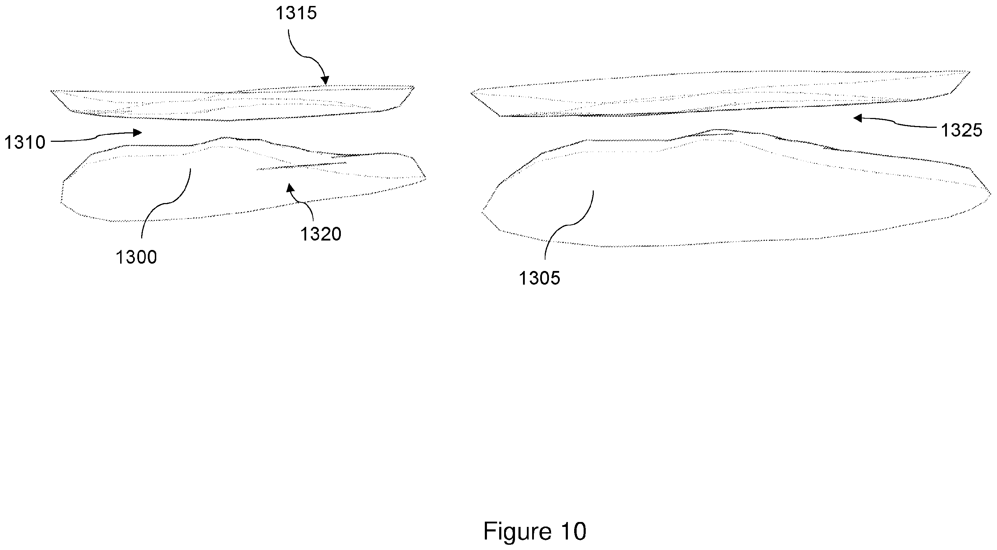

To illustrate the above grouping, Figure 10 shows smaller 1300 and larger 1305 statistical shape models of the analysis of lumbar L1-2 and L2-3 (subset of all lumbar levels) intervertebral body spaces suitable for lateral interbody device design. The interbody space 1310 is defined by superior 1315 and inferior 1320 endplates.

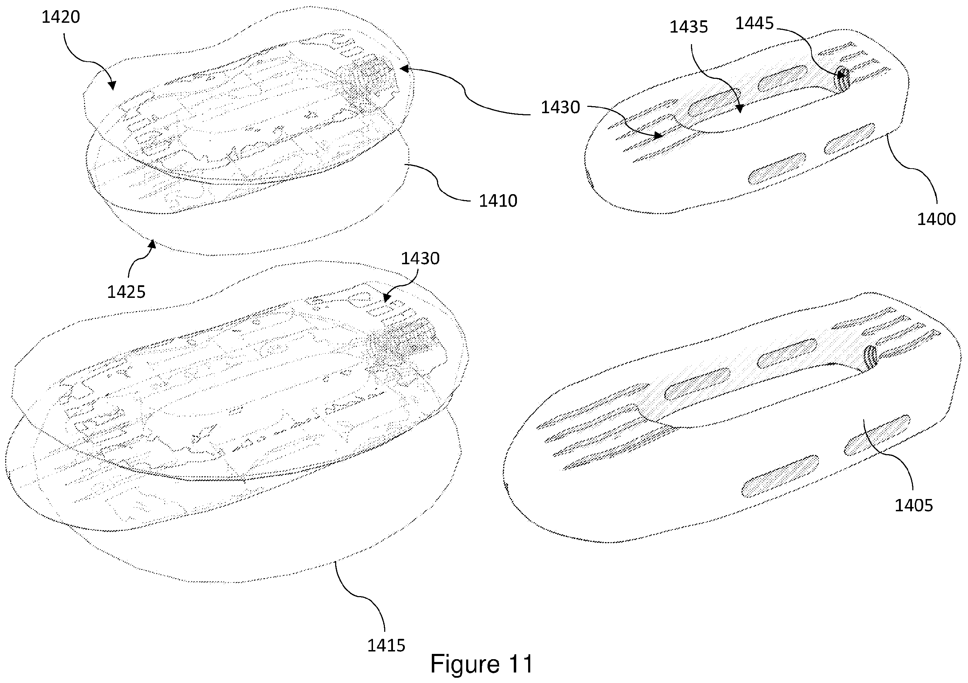

Figure 11 illustrates an anterior-superior-left view of smaller 1400 and larger 1405 lateral lumbar interbody fusion devices corresponding to size and shape of the smaller 1410 and larger 1415 interbody spaces (as shown in Figure 10 as 1310 and 1325). The interbody (negative) spaces used to design the devices are defined by superior 1420 and inferior 1425 endplate statistical shape models. The smaller 1400 and larger 1405 devices have anatomy contacting surfaces shaped to fit within the interbody spaces, resulting in greater contact surface area between endplate and devices including in the areas with anti-expulsion teeth 1430. That is, the models associated with devices 1400, 1405 are warped from an initial shape to a target shape, the target shape accommodating greater contact surface area between the endplates and devices 1400, 1405. The initial shape associated with the models of the devices 1400, 1405 may be relatively flat. A warping process is further described in US Publication No. 20220226130. The lateral interbody device designs shown here also feature graft windows 1435 and insertion instrumentation interface (threaded hole 1445)The device designs also feature allometrically scaling contact surface areas.

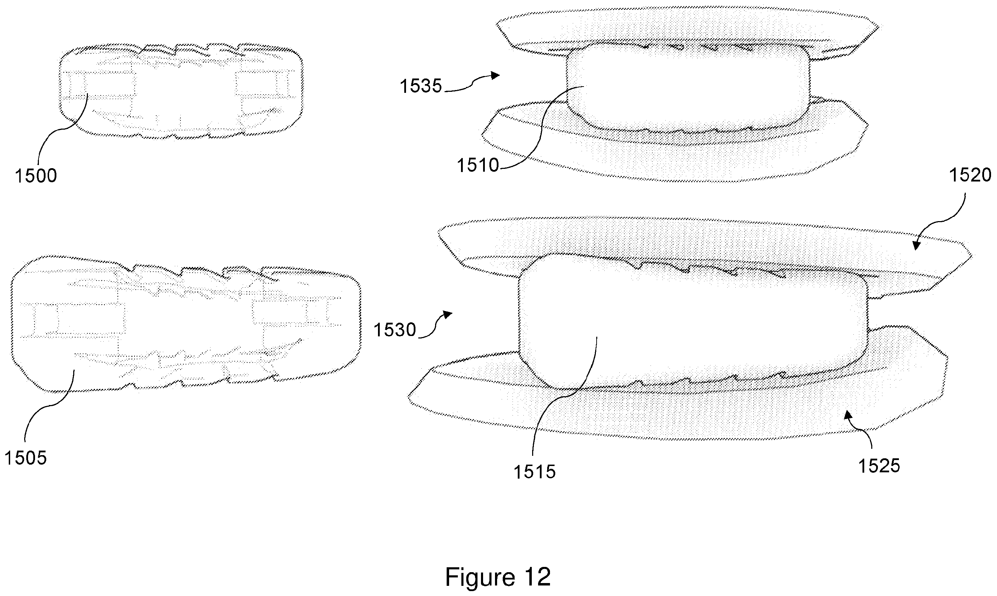

With the above in mind, Figure 12 illustrates a sagittal plane (left) view of smaller 1500 and larger 1505 lateral approach type interbody fusion devices. The smaller 1510 and larger 1515 devices are shown in the smaller 1535 and larger 1530 interbody (negative) spaces defined by the superior 1520 and inferior 1525 endplate statistical shapes. Note that as well as the size change between smaller 1500 and larger 1505 interbody cages, the statistical shape model includes angle differences.

Overall, if an aim is to create a shape space that can be used to design anterior approach lumbar devices (such as ALIF fusion cages, or joint replacement devices), the L3-4, L4-5 and L5-S1 subset of the dataset may be used to define the statistical shape models to control the device requirements and 3D shape.

At step 30 in Figure 1, the device design may be varied to achieve other desired device parameters / design outcomes. In other words, other device parameters may be included to define the one or more surfaces of the medical implant. For example, in cervical interbody instrumentation, surgeons typically aim to distract the interbody space (restore loss of height) and restore normal interbody angle. The anterior device height is also partially governed by the need to interface with insertion or other instrumentation, such as screws, that secure the device in place. These minimum requirements can be applied to the statistical shapes and thereby used as design constraints for the devices.

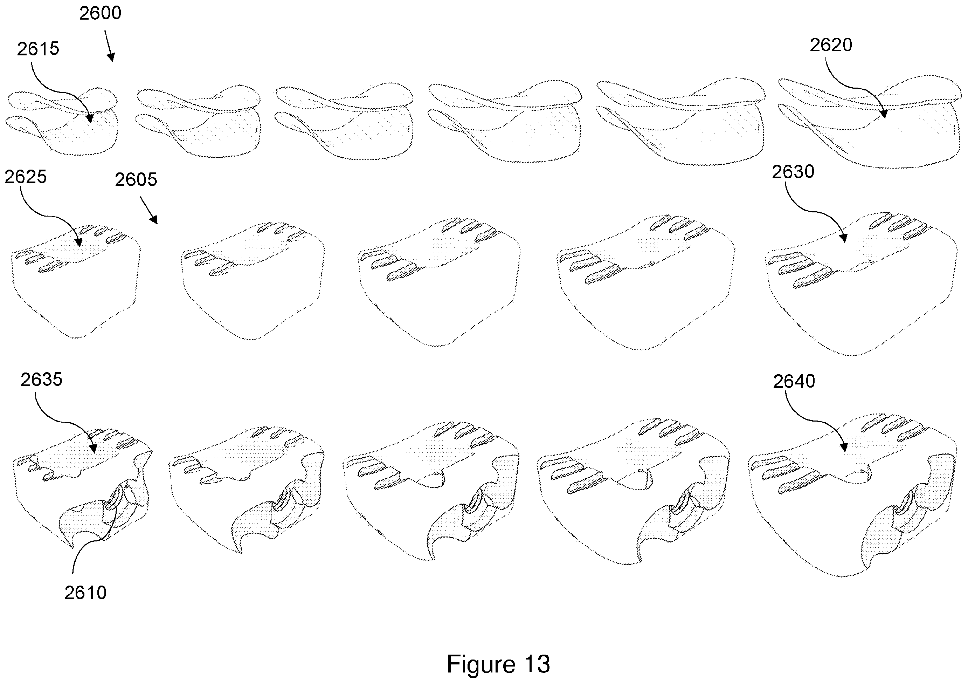

By way of example, Figure 13 illustrates cervical interbody statistical shapes 2600 incorporating endplate allometric shapes as well as minimum posterior and anterior heights and minimum angles between the endplates. Statistical interbody/endplate/negative space shapes vary from small 2615 to large 2620 and contain both size and size correlated (allometric) shape variation. These negative spaces can then be used to create interbody device shapes 2605 ranging from small 2625 to large 2630 that incorporate both allometric shape, as well as parameter (width, surface area) characteristics (as shown in Figure 4). As part of forming the interbody device shapes 2605, an initial shaped may be warped to a target shape. The target shapes provide suitable / maximum contact with the statistical shapes (to accommodate small to large people). The allometric interbody device shapes can then have features such as integral screw holes and/or insertion instrumentation interfaces 2610 added, depending on the final device functional requirements.

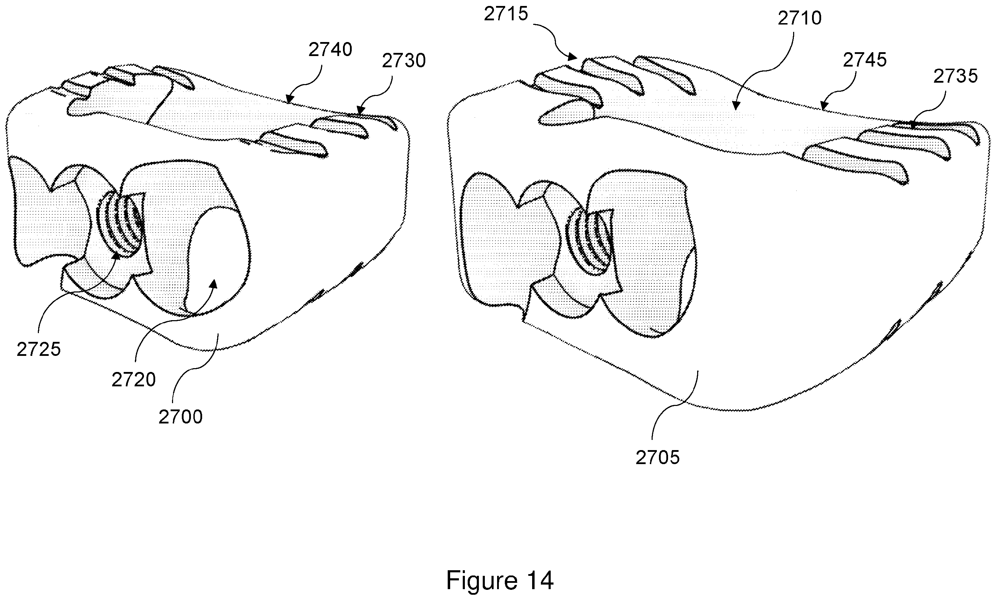

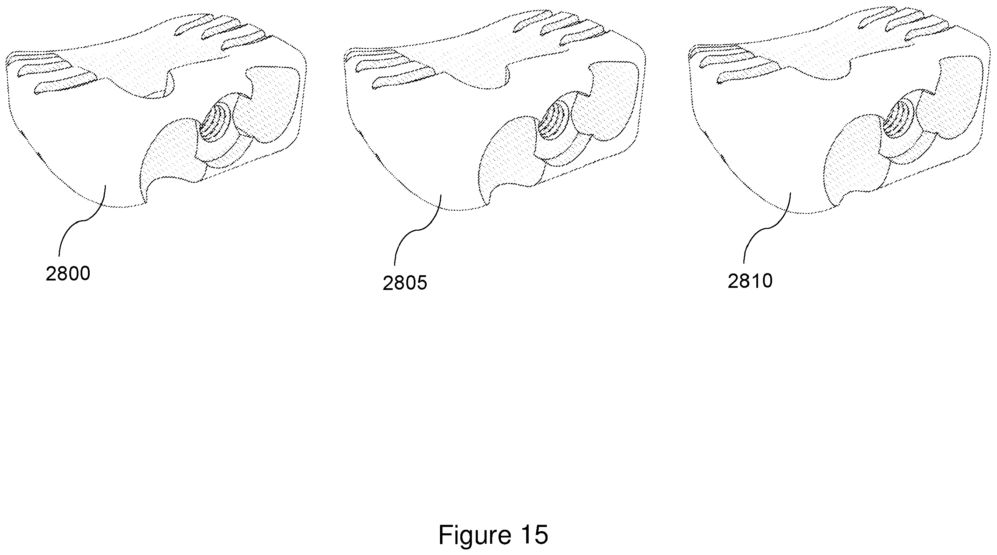

In a similar manner, Figure 14 illustrates small 2700 and large 2705 interbody device designs incorporating allometric parameter (width, contact surface area) and shape characteristics. Additional device features including a graft window 2710, anti-expulsion teeth 2715, holes for integral screws 2720, threaded interface for insertion instrumentation 2725 can be added once the shape has been defined in steps 10 and 20. In this regard, from steps 10 and 20, note that there are differences in the width, surface area and curvatures of the device-anatomy interface between small 2730 and large 2735 designs. There are also differences in curvatures at the device-anatomy interface of the posterior device wall of the small 2740 compared to large 2745 devices. In Figure 15, height and angle variations for an allometric interbody cage design are shown with 2800 being a minimum (small height, minimum angle), 2805 being a medium (medium height, medium angle) and 2810 being the largest (high height, highest angle).

The combination of statistical shape modelling with device parameters (such as minimum anterior and posterior heights and/or angles) can be used to dictate final negative space shapes (including angles) that determine the final device shapes. This approach can be useful when there are minimum or target parameters that are typically used. In this regard, the device parameters can be separate from the associated data of the statistical shape modelling.

For example, in spinal interbody instrumentation, surgeons typically aim to distract the interbody space to restore loss of height and restore normal interbody angle. In such cases, device parameters may be obtained through surgeon specifications / preferences. For instance, a surgeon may require a predetermined spinal interbody posterior height and angle. The combination of minimum posterior height and angle will then determine the anterior height.

Other device parameters may be obtained through specification values published in the academic / scientific peer reviewed literature. For example, studies may report an average value for normal posterior, anterior, and/or angle for a particular interbody space in healthy spines. These parameters may be used as target values when designing the devices. Devices designed with these parameters will restore pathological interbody spaces to normal alignments. This can aid in restoration of relative positioning of adjacent vertebrae, as well as overall alignment of the spine, which is important in restoring and maintaining balance (for example sagittal balance, or lordosis).

The anterior device height is also partially governed by the need to interface with insertion or other instrumentation, such as screws, that secure the device in place. These minimum requirements can be applied to the statistical shapes and thereby used as design constraints for the devices.

Other design requirements/parameters may be obtained from testing standards that the devices must pass to gain regulatory approval to be marketed. For instance, spinal interbody posterior minimum height is effectively set by the requirements set out in ASTM F2077. Other standards may include ASTM or ISO to set certain parameters. Combinations of minimum anterior and posterior heights then set minimum angles between statistical shape representations of the endplates that define that negative spaces used to create the device shapes. Other angles are possible, but this defines one set of angles that need to be in the device range.

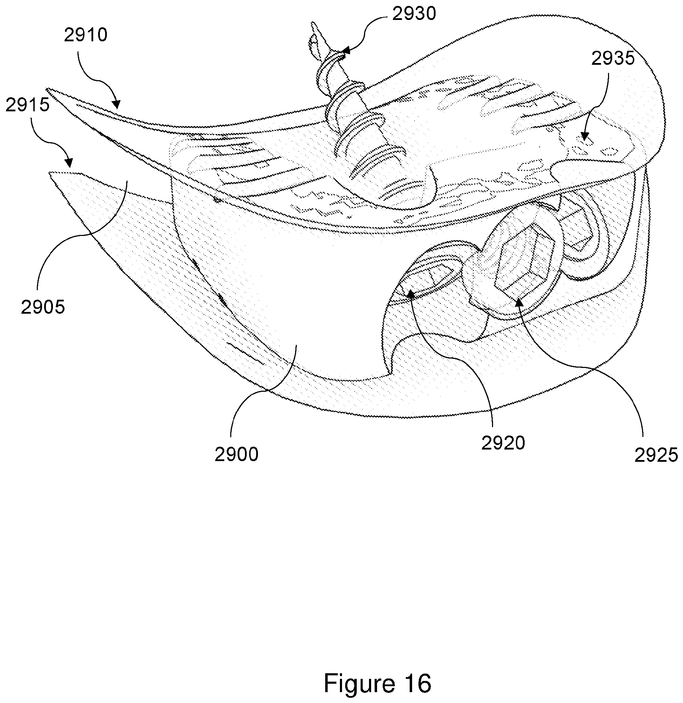

An initial minimum parameter (minimum posterior and anterior heights defining minimum angle) device shape range can be expanded by including additional height and angle options for each device footprint. Such methods can create devices that fit very well with the statistical shapes defined by the method. For example, Figure 16 illustrates an allometric interbody device 2900 in an interbody space 2905 defined by superior 2910 and inferior 2915 endplate statistical shapes. The device is shown with a fastening portion include integral screws 2920 and locking cap 2925, with the integral screw shown penetrating through the superior endplate 2930. Note the good contact 2935 between endplate statistical shapes and the allometric device design, showing that the allometric device design fits well with the statistical interbody space shape.

Overall, once a suitable proportion of shape variance has been identified on one or more axes, the shape space can be used to create a range of statistical shapes capturing this proportion of shape variance at discrete intervals. These statistical shapes can be used to design better fitting generic devices as they (the statistical shapes) capture the complex 3D shape characteristics of the anatomical structures that the device will interface with more fully than traditional parametric measurements can. Final device designs can be achieved in a number of different ways from the statistical shape models. They can be derived or built directly from them. For example, a plate outline can be drawn on a statistical shape model, the surface of the shape model defined by the outline can be extruded to give the plate thickness. This method would create a plate that conforms to the surface of statistical shape. The final device design could be achieved through computer aided design (CAD) operations, adding features such as chamfers, fillets, holes, threads, instrumentation interfaces etc. Alternatively, uniform device templates can be adapted to fit the statistical shapes. This method can include more, or potentially all, of the final device design features or can be used to create a close-to-complete device to which final features are added. Furthermore, it would also be appreciated that artificial intelligence techniques (including machine learning) could be applied to any one of the steps in 10, 20 or 30 to achieve the implant designs. That is, the data and parameters driving the design through steps 10, 20 and/or 30 may be performed through a machine learning model that finds suitable compromises in the implant designs. Machine learning processes may also be applied to refine the shape space. For example, the ellipsoid shape space shown in Figures 7 and 8 could be altered as a result of machine learning processes, to reduce further then number of discrete points (1035) used to create devices.

Once the final implant design(s) are established from steps 10 to 30, at step 40, the medical implant(s) are produced based on these final design(s). As the implants are generic devices, they can be physically manufactured through more traditional manufacturing techniques, such as casting and machining (as opposed to additive manufacturing etc.). However, it will be appreciated that more advanced manufacturing techniques (eg, additive manufacturing) may be adopted if the implant shapes are complex, or if the final implants at step 40 contain complex geometric features such as lattice structures.

Furthermore, the medical implants are typically packaged in a sterile manner until they are required for surgery. Once a surgeon has gauged which implant will be most suitable from a set/system of implants, the packaging for the implant is removed and then the implant is inserted into the patient.

The method 1 provides an advantageous way of using data derived from anatomical database to design a range of generic (off-the-shelf) implantable medical devices. The implants provide a much better fit compared to current generic devices that fail to appreciate, for example, the allometric relationships between different size patients. The method 1 has broad applications and can, for instance, maintain a constant output (eg, stress / pressure at the anatomy-device interface) by varying shape requirements (eg, contact surface area) so that the device has a constant performance across a different size range. Furthermore, using data from specific regions, or level specific shape analyses to define statistical shapes for use in designing region specific devices, allows for more refined designs to be created.

The use of CI regions in the models, together with selecting a cohort of devices that will suitable service the CI, also provides a commercially feasible plan in implementing a set of implants. That is, an exorbitant amount of implants are not required to service the population - rather the designs are selected based on sampling the CI uniformly at discrete points. To this end, the sampling of these CI is made easier from, for instance, the combination of N-dimensional shape modes being suitably modelled to cover the majority of shapes observed in the population. Discrete shapes can be derived at intervals within the defined shape space and used to form the discrete 3D device shapes making up the device range.

Separately, the combination of statistical shape modelling with other device parameters (such as minimum anterior and posterior heights) dictate negative space shapes (including angles) that determine the end device shapes. This combination of modelling makes the end implant more suitable for surgery. For example, minimum anterior height is governed by interfacing instruments such as inserters and integral screws etc. This approach also makes the implant devices suitable for passing regulatory testing standards. For example, minimum posterior height (in Anterior Cervical Discectomy and Fusion, or ACDF, devices at least) is governed by ASTM F2077.

In this specification, adjectives such as first and second, left and right, top and bottom, and the like may be used solely to distinguish one element or action from another element or action without necessarily requiring or implying any actual such relationship or order. Where the context permits, reference to an integer or a component or step (or the like) is not to be interpreted as being limited to only one of that integer, component, or step, but rather could be one or more of that integer, component, or step etc.

In this specification, the terms 'comprises', 'comprising', 'includes', 'including', or similar terms are intended to mean a non-exclusive inclusion, such that a method, system or apparatus that comprises a list of elements does not include those elements solely, but may well include other elements not listed.

## Claims

1. A method (1) including:
using a model to assist in defining one or more outer surfaces of a medical implant (10), the model being based on a statistical analysis of data, associated with an intended location of a medical implant, from multiple patients; and
producing the medical implant based on the one or more outer surfaces,
wherein:
the data includes information associated with patients of different sizes; and
the step of using the model includes retrieving a statistical shape based on the statistical analysis of the data;
the statistical shape includes at least two surfaces and a space therebetween that assists in defining the one or more surfaces of the medical implant.

2. The method (1) of claim 1, wherein the step of using the model includes defining the one or more outer surfaces of a medical implant based on the statistical shape.

3. The method (1) of claim 1 or 2, wherein the step of using the model includes using the statistical relationship established from the data to retrieve a statistical shape associated with the intended location of the medical implant.

4. The method (1) of claim 3, wherein the statistical relationship includes an equation relating to a line of best fit (205) through the data.

5. The method (1) of claim 3 or 4, wherein the statistical relationship includes size related, including isometric or allometric, scaling.

6. The method (1) of any one of the preceding claims, wherein the statistical analysis of the data is in more than two dimensions.

7. The method (1) of any one of the preceding claims, wherein a matrix decomposition is performed on the data as part of developing the model.

8. The method (1) of any one of the preceding claims, wherein the dimensionality of the data is reduced into a smaller subset.

9. The method (1) of any one of the preceding claims, wherein the statistical analysis includes creating an n-dimensional shape space.

10. The method (1) of any one of the preceding claims, wherein using the model includes determining a confidence interval of data within a defined criteria.

11. The method of claim 10, wherein the defined criteria is defined in a manner to create a suitable cohort of medical implants.

12. The method (1) of any one of the preceding claims, wherein the method further comprises including one or more device parameters to assist in defining the one or more outer surfaces of the medical implant, the one or more device parameters being based on one or more predetermined parameters set by an external specification.

13. The method (1) of any one of the preceding claims, wherein the intended location includes a location associated with two adjacent vertebral bodies.

14. A medical implant comprising:
a body having:
one or more outer surfaces configured to engage with an intended location of the medical implant,
wherein:
the one or more outer surfaces are based on a statistical analysis of data:
associated with the intended location of the medical implant; and
from multiple patients of different sizes; and
the one or more outer surfaces are established from a statistical relationship derived from the data whereby the statistical relationship allows a statistical shape to be established, the statistical shape includes at least two surfaces and a space therebetween that assists in defining the one or more outer surfaces of the medical implant.

15. A medical implant system including:
multiple medical implants having one or more outer surfaces configured to engage with an intended location of each medical implant,
wherein:
the one or more outer surfaces are based on a statistical analysis of data:
associated with the intended locations of the medical implants; and
from multiple patients of different sizes; and
the one or more outer surfaces are established from a statistical relationship derived from the data whereby the statistical relationship allows a statistical shape to be established, the statistical shape includes at least two surfaces and a space therebetween that assists in defining the one or more outer surfaces of the medical implant.

## Patentansprüche

1. Ein Verfahren (1), das Folgendes umfasst:
Verwenden eines Modells, um beim Definieren einer oder mehrerer äußerer Oberflächen eines medizinischen Implantats (10) zu helfen, wobei das Modell auf einer statistischen Analyse von Daten, die mit einem beabsichtigten Ort eines medizinischen Implantats assoziiert sind, von mehreren Patienten basiert; und
Herstellen des medizinischen Implantats basierend auf der einen oder den mehreren äußeren Oberflächen, wobei:
die Daten Informationen umfassen, die mit Patienten unterschiedlicher Größen assoziiert sind; und
der Schritt des Verwendens des Modells das Abrufen einer statistischen Form basierend auf der statistischen Analyse der Daten umfasst;
die statistische Form mindestens zwei Oberflächen und einen Raum dazwischen umfasst, der beim Definieren der einen oder der mehreren Oberflächen des medizinischen Implantats hilft.

2. Verfahren (1) gemäß Anspruch 1, wobei der Schritt des Verwendens des Modells das Definieren der einen oder der mehreren äußeren Oberflächen eines medizinischen Implantats basierend auf der statistischen Form umfasst.

3. Verfahren (1) gemäß Anspruch 1 oder 2, wobei der Schritt des Verwendens des Modells das Verwenden der statistischen Beziehung, die aus den Daten erstellt wurde, umfasst, um eine statistische Form abzurufen, die mit dem beabsichtigten Ort des medizinischen Implantats assoziiert ist.

4. Verfahren (1) gemäß Anspruch 3, wobei die statistische Beziehung eine Gleichung umfasst, die sich auf eine Ausgleichsgerade (205) durch die Daten bezieht.

5. Verfahren (1) gemäß Anspruch 3 oder 4, wobei die statistische Beziehung größenbezogene Skalierung, einschließlich isometrischer oder allometrischer Skalierung, umfasst.

6. Verfahren (1) gemäß einem der vorhergehenden Ansprüche, wobei die statistische Analyse der Daten in mehr als zwei Dimensionen erfolgt.

7. Verfahren (1) gemäß einem der vorhergehenden Ansprüche, wobei eine Matrixzerlegung an den Daten als Teil der Entwicklung des Modells durchgeführt wird.

8. Verfahren (1) gemäß einem der vorhergehenden Ansprüche, wobei die Dimensionalität der Daten auf eine kleinere Teilmenge reduziert wird.

9. Verfahren (1) gemäß einem der vorhergehenden Ansprüche, wobei die statistische Analyse das Erzeugen eines n-dimensionalen Formraums umfasst.

10. Verfahren (1) gemäß einem der vorhergehenden Ansprüche, wobei das Verwenden des Modells das Bestimmen eines Konfidenzintervalls von Daten innerhalb eines definierten Kriteriums umfasst.

11. Verfahren gemäß Anspruch 10, wobei das definierte Kriterium auf eine Weise definiert ist, um eine geeignete Kohorte von medizinischen Implantaten zu erzeugen.

12. Verfahren (1) gemäß einem der vorhergehenden Ansprüche, wobei das Verfahren ferner das Einbeziehen eines oder mehrerer Vorrichtungsparameter beinhaltet, um beim Definieren der einen oder der mehreren äußeren Oberflächen des medizinischen Implantats zu helfen, wobei der eine oder die mehreren Vorrichtungsparameter auf einem oder mehreren vorbestimmten Parametern basieren, die durch eine externe Spezifikation festgelegt sind.

13. Verfahren (1) gemäß einem der vorhergehenden Ansprüche, wobei der beabsichtigte Ort einen Ort umfasst, der mit zwei benachbarten Wirbelkörpern assoziiert ist.

14. Ein medizinisches Implantat, das Folgendes beinhaltet:
einen Körper, der Folgendes aufweist:
eine oder mehrere äußere Oberflächen, die konfiguriert sind, um mit einem beabsichtigten Ort des medizinischen Implantats in Eingriff zu kommen,
wobei:
die eine oder die mehreren äußeren Oberflächen auf einer statistischen Analyse von Daten basieren:
die mit dem beabsichtigten Ort des medizinischen Implantats assoziiert sind; und
von mehreren Patienten unterschiedlicher Größen; und
die eine oder die mehreren äußeren Oberflächen aus einer statistischen Beziehung erstellt werden, die aus den Daten abgeleitet ist, wodurch die statistische Beziehung das Erstellen einer statistischen Form ermöglicht, wobei die statistische Form mindestens zwei Oberflächen und einen Raum dazwischen umfasst, der beim Definieren der einen oder der mehreren äußeren Oberflächen des medizinischen Implantats hilft.

15. Ein medizinisches Implantatsystem, das Folgendes umfasst:
mehrere medizinische Implantate, die eine oder mehrere äußere Oberflächen aufweisen, die konfiguriert sind, um mit einem beabsichtigten Ort jedes medizinischen Implantats in Eingriff zu kommen,
wobei:
die eine oder die mehreren äußeren Oberflächen auf einer statistischen Analyse von Daten basieren:
die mit den beabsichtigten Orten der medizinischen Implantate assoziiert sind; und
von mehreren Patienten unterschiedlicher Größen; und
die eine oder die mehreren äußeren Oberflächen aus einer statistischen Beziehung erstellt werden, die aus den Daten abgeleitet ist, wodurch die statistische Beziehung das Erstellen einer statistischen Form ermöglicht, wobei die statistische Form mindestens zwei Oberflächen und einen Raum dazwischen umfasst, der beim Definieren der einen oder der mehreren äußeren Oberflächen des medizinischen Implantats hilft.

## Revendications

1. Un procédé (1) incluant :
l'utilisation d'un modèle pour aider à la définition d'une ou de plusieurs surfaces externes d'un implant médical (10), le modèle étant fondé sur une analyse statistique de données, associées à un emplacement prévu d'un implant médical, provenant de multiples patients ; et
la production de l'implant médical sur la base des une ou plusieurs surfaces externes, dans lequel :
les données incluent des informations associées à des patients de différentes tailles ; et
l'étape d'utilisation du modèle inclut la récupération d'une forme statistique sur la base de l'analyse statistique des données ;
la forme statistique inclut au moins deux surfaces et un espace entre elles qui aide à la définition des une ou plusieurs surfaces de l'implant médical.

2. Le procédé (1) de la revendication 1, dans lequel l'étape d'utilisation du modèle inclut la définition des une ou plusieurs surfaces externes d'un implant médical sur la base de la forme statistique.

3. Le procédé (1) de la revendication 1 ou de la revendication 2, dans lequel l'étape d'utilisation du modèle inclut l'utilisation de la relation statistique établie à partir des données pour récupérer une forme statistique associée à l'emplacement prévu de l'implant médical.

4. Le procédé (1) de la revendication 3, dans lequel la relation statistique inclut une équation relative à une droite de meilleur ajustement (205) à partir des données.

5. Le procédé (1) de la revendication 3 ou de la revendication 4, dans lequel la relation statistique inclut une mise à l'échelle liée à la taille, notamment isométrique ou allométrique.

6. Le procédé (1) de l'une quelconque des revendications précédentes, dans lequel l'analyse statistique des données est sur plus de deux dimensions.

7. Le procédé (1) de l'une quelconque des revendications précédentes, dans lequel une décomposition matricielle est effectuée sur les données dans le cadre du développement du modèle.

8. Le procédé (1) de l'une quelconque des revendications précédentes, dans lequel la dimensionnalité des données est réduite à un sous-ensemble plus restreint.

9. Le procédé (1) de l'une quelconque des revendications précédentes, dans lequel l'analyse statistique inclut la création d'un espace de forme à n dimensions.

10. Le procédé (1) de l'une quelconque des revendications précédentes, dans lequel l'utilisation du modèle inclut la détermination d'un intervalle de confiance de données au sein d'un critère défini.

11. Le procédé de la revendication 10, dans lequel le critère défini est défini de manière à créer une cohorte appropriée d'implants médicaux.

12. Le procédé (1) de l'une quelconque des revendications précédentes, le procédé comprenant en outre l'inclusion d'un ou de plusieurs paramètres de dispositif pour aider à la définition des une ou plusieurs surfaces externes de l'implant médical, les un ou plusieurs paramètres de dispositif étant fondés sur un ou plusieurs paramètres prédéterminés fixés par une spécification externe.

13. Le procédé (1) de l'une quelconque des revendications précédentes, dans lequel l'emplacement prévu inclut un emplacement associé à deux corps vertébraux adjacents.

14. Un implant médical comprenant :
un corps présentant :
une ou plusieurs surfaces externes configurées pour s'engager avec un emplacement prévu de l'implant médical,
dans lequel :
les une ou plusieurs surfaces externes sont fondées sur une analyse statistique de données :
associées à l'emplacement prévu de l'implant médical ; et
provenant de multiples patients de différentes tailles ; et
les une ou plusieurs surfaces externes sont établies à partir d'une relation statistique dérivée des données, moyennant quoi la relation statistique permet d'établir une forme statistique, la forme statistique inclut au moins deux surfaces et un espace entre elles qui aide à la définition des une ou plusieurs surfaces externes de l'implant médical.

15. Un système d'implants médicaux incluant :
de multiples implants médicaux présentant une ou plusieurs surfaces externes configurées pour s'engager avec un emplacement prévu de chaque implant médical, dans lequel :
les une ou plusieurs surfaces externes sont fondées sur une analyse statistique de données :
associées aux emplacements prévus des implants médicaux ; et
provenant de multiples patients de différentes tailles ; et
les une ou plusieurs surfaces externes sont établies à partir d'une relation statistique dérivée des données, moyennant quoi la relation statistique permet d'établir une forme statistique, la forme statistique inclut au moins deux surfaces et un espace entre elles qui aide à la définition des une ou plusieurs surfaces externes de l'implant médical.
